# EUROPEAN PATENT SPECIFICATION

(11) **EP 0 478 549 B1**
(45) Date of publication and mention of the grant of the patent: **16.09.1998**
(21) Application number: 90903978.6
(22) Date of filing: 16.02.1990
(51) Int. Cl.: C07K 2/00, C12P 21/00, A61K 35/74

(54) **PRODUCTS FOR INHIBITING THE ADHESION, GROWTH AND/OR SURVIVAL OF PATHOGENS**
ERZEUGNISSE ZUR INHIBIERUNG DER BINDUNG, DES WACHSENS UND/ODER DES ÜBERLEBENS VON PATHOGENEN
PRODUITS SERVANT A EMPECHER L'ADHESION, LA CROISSANCE ET/OU LA SURVIE D'AGENTS PATHOGENES

(30) Priority: 17.02.1989 SE 8900546
(43) Date of publication of application: 08.04.1992
(73) Proprietor: Arla, ek. för., 105 46 Stockholm (SE)
(72) Inventor: CONWAY, Patricia, S-414 76 Göteborg (SE); KJELLEBERG, Staffan, S-414 76 Göteborg (SE)
(74) Representative: Perneborg, Henry T.
(86) International application number: SE9000105
(87) International publication number: WO9009398

(56) References cited:
- EP-A- 33 584
- EP-A- 203 586
- US-A- 4 767 623
- INFECTION AND IMMUNITY, Vol. 49, No. 3, 1985 KAZUOKI ISHIHARA et al: "Growth Inhibition of Streptococcus mutans by Cellular Extracts of Human Intestinal Lactic Acid Bacteria", see page 692 - page 694.

## Description

The present invention concerns products for inhibiting the adhesion of pathogens to intestinal epithelial mucosa, or for inhibiting the growth and/or survival of pathogens in animals including humans.

The invention also concerns a method for producing said products and preparations containing the products. Furthermore, the invention concerns preparations containing the products or viable strains of certain Lactobacillus as well as a method of treating animals with the claimed products.

The products are Lactobacillus metabolites, including high molecular weight proteinaceous compounds, which inhibit the adhesion, growth and/or survival of pathogens, such as Escherichia coli, Clostridium, Salmonella, Campylobacter and Streptococcus strains.

The role of Lactobacillus within the gastrointestinal tract has been extensively investigated and reviewed (e.g. Sandine et al, 1972; Speck, 1976; Sandine, 1979; Alm, 1980; Pollmann et al, 1980; Shahani and Ayebo, 1980; Fernandes et al, 1987) and a number of beneficial roles have been proposed (see Table 1).

**Table 1**

| Summary of the reported beneficial effects of lactobacillus in the gastrointestinal tract. Numbers in parenthesis refer to examples of studies for each area. |
|---|
| Establishment and stabilization of the gut microflora (1) |
| Protection against pathogen colonization (2) |
| Reduces bacterial diarrhea and subsequent mortality (3) |
| Production of bacteriocins/antimicrobial compounds (4) |
| Increased weight gain for a range of animal hosts (5, 6) |
| Stimulation of the immune system (7) |
| Decreased incidence of tumours (8) |
| Decreased risk for colon cancer (9) |
| Assimilation of cholesterol (10) |
| Reduction of serum cholesterols (11) |
| Deconjugation of bile acids (12) |
| Improved gastrointestinal motility in the elderly (13) |
| Improved egg production and quality in hens (14) |
| Improved nutritional status of food (15) |
| Improved tolerance of lactose (16) |
| Metabolism of some drugs (17) |
| 1. Morotomi et al, 1975; 2. Muralidhara et al, 1977; 3. Luckey, 1984; 4. Barefoot and Klaenhammer, 1983; 5. Robinson and Thompson, 1952; 6. Lessard and Brisson, 1987; 7. Perdigon et al, 1987; 8. Goldin and Gorbach, 1980; 9. Goldin et al, 1980; 10. Gunewald, 1982; 11. Gilliland et al, 1985; 12. Gilliland and Speck, 1977; 13. Alm et al, 1983; 14. Miles et al, 1981; 15. Gurr, 1987; 16. Kolars et al, 1984; 17. Pradham and Majumdar, 1986. |

For all the studies presenting the benficial role of the entire normal microflora, there have been almost as many studies implicating lactobacilli as having the capacity to achieve similar beneficial effects. Clearly, the validity of such a situation raises doubt in the minds of many. Doubts have been strengthened by contradictory or non-conclusive results as to the beneficiac effects of lactobacilli (e.g. as summarized by Tuschy, 1986).

Non-conclusive or not statistically significant results are often reported when attempts are made to confirm that lactobacilli improve the health of the host. Inconsistencies in reports began at the turn of the century, when the works of Cohendy (1906a and b) and Belonovsky (1907) showing colonization of the intestine were not supported by other studies in which the administered strain could not be estabilished (Luerssen and Kühn, 1908; Herter and Kendall, 1908; Distaso and Schiller, 1914; Hull and Rettger, 1914). This situation continues to occur today as many workers report a failure in persistence of administered lactobacilli (e.g. Jonsson, 1986; Pedersen and Tannock, 1986) while others have succeeded in achieving implantation in the same host (Conway et al, manuscript). Contradictory results appear for all reported beneficial effects. For example, Muralidhara et al (1977) reported a decrease in E. coli in lactobacilli dosed pigs while Pollmann et al (1980) showed no difference in E. coli levels between control and lactobacilli dosed piglets. These types of results are assumed to be comparable without consideration that lactobacilli strains and conditions of the host may vary markedly.

It has been convincingly shown that lactobacilli are beneficial for the health of newborn babies (e.g. Yoshioka et al, 1983) as suggested for fowls (Fuller, 1973), and that they reduce pathogenic coloform levels (e.g. Barrow et al, 1980). A common statement in the introduction of papers dealing with lactobacilli in the gastrointestinal tract is: Lactobacfilli are known to regulate the intestinal microflora and influence the health and well being of the host. This poorly defined statement is not entirely supported by published data. Within the adult alimentary tract, the clear definition of the role of indigenous lactobacilli has been clouded by the issue of whether ingested lactobacilli play a beneficial role, in some cases without destinguishing that the lactobacilli used to produce fermented milk products may have entirely different characteristics from those colonizing the gastrointestinal tract. There is a very obvious trend that lactobacilli contribute significantly to the well being of the host (reviews cited previously), however, this has often been reflected as trends rather than conclusive facts and reports often differ.

### INDIGENOUS LACTOBACILLI OF THE GASTROINTESTINAL ECOSYSTEM

### Description of the Ecosystem

The gastrointestinal tract of man and most animals is a complex ecosystem in which a dynamic equilibrium exists between the microflora, the diet and the host (Savage, 1977). Contributions to the ecosystem from the host include the peristalsis and motility, the immune response, the mucosal surface and secretions. The microflora consists of extremely diverse bacterial species colonizing both lumenal contents and epithelia surfaces. Nisse (1916) was the first to propose that the intestinal microflora may yield protection against infection and Freter (1955, 1956) provided direct evidence that elimination of the flora rendered animals susceptible to pathogens. While it has been shown by many that the normal microflora play a protection role (reviewed by Hentges, 1983), the specific contribution of the lactobacilli is more diffuse. In the nwe-born, lactobacilli constitute the major bacterial genus prior to the successive colonization by other bacterial species from the environment and the mother (e.g. Bettelheim et al, 1974; Ducluzeau, 1983). Using conventional and gnotobiotic animals, Morotomi et al (1975) showed that lactobacilli control the population levels of bacteria in the stomach and small intestines of rats (Morotomi et al, 1975). Robinson and Thompson (1952) reported that breast fed infants developed a stable microflora of about 99% Lactobacillus bifidus within 3 to 4 days and that these lactobacilli lowered the incidence of colic and digestive disturbances. The introduction of cow's milk has been shown to decrease the numbers of L. bifidus and increase the numbers of E. coli by 1-2 and 2-3 log counts, respectively (Willis et al, 1973). Ingestion of solid food induces the rapid development of the complex microflora, of which Lactobacillus represents one of the dominant groups.

The average levels of lactobacilli cultured from samples through the alimentary tract are shown in Table 2. While the data for humans reflects the lumenal contents profile, numerous animal studies have demonstrated that Lactobacillus colonized the squamous epithelia of a range of hosts including chickens (Fuller and Turvey, 1971), swine (Tannock and Smith, 1970) and rodents (Brownlee and Moss, 1961). Permanent colonization of the human epithelium has not been examined to date, however, lactobacilli have been cultured from surgical samples from the mucosa of the jejunum (Plaut et al, 1967; Nelson and Mata, 1970) and colon (Nelson and Mata, 1970; Edmiston et al, 1982). These studies, supported by the demonstration of in vitro adhesion to human ileal cells (Conway et al, 1987) and to fetal intestinal cells (Kleeman and Klaenhammer, 1982), is suggestive that colonization of the healthy human mucosa may occur.

**Table 2**

| Occurrence of lactobacilli in the gastrointestinal tract of man and pigs. Values expressed as log bacteria per ml or gram of contents and per 100 mm² epithelial surface. | | | | | |
|---|---|---|---|---|---|
| Host | Stomach | | Ileum | | Colon |
| | Surface | Contents | Surface | Contents | |
| Man^{a} | ND | 0-4 | ND | 3-7 | 4-8 |
| Rats^{b} | 3-4 | ND | ND | ND | 7-9 |
| Pigs^{c} | 7.9 | 8.9 | 6.9 | 8.3 | 9.6 |

| | | | | | |
|---|---|---|---|---|---|
| a. Data complied from Evaldson et al (1982) | | | | | |
| b. Data complied from Adams and Conway (1981) | | | | | |
| c. Data complied from Conway et al (manuscript in preparation | | | | | |
| ND corresponds to no available data | | | | | |

### Stability

During development in the young, the normal microflora is rather unstable and the host therefore more susceptible to invasion by ingested pathogens. In contrast, the normal human adult microflora has been shown to be remarkably stable (reviewed by Hentges, 1983), although this may reflect difficulties of sampling and culturing, because changes have only largely been observed for the easily recognized and cultured bacterial types (Tannock, 1983). Consequently, lactobacilli have been shown to be sensitive to change, for example, lactobacilli are suppressed very rapidly in man when the subject is exposed to nervous-emotional stress effects, as provoked prior to cosmic flights (Lizko et al, 1984). Furthermore, piglets when being weaned onto solid foods are extremely susceptible to bacterial induced diarrhea from E. coli K88 (e.g. Tziperi et al, 1984).

From numerous animal dietary studies, the lactobacilli emerge as indicators of dietary stress (reviewed by Tannock, 1983). Mice fed a semisynthetic diet had fewer faecal lactobacilli than controls (Dubos and Schaedler, 1962) and rats fed a meat diet (Brownlee and Moss, 1961), a synthetic corn oil diet (Brockett and Tannock, 1981) or dietary additives (Adams, 1980; Adams and Conway,. 1981) had no lactobacilli remaining adhered to the squamous epithelium. In addition, rodents had fewer lactobacilli in the stomach and intestine after several days of starvation (Porter nad Rettger, 1940; Morishita and Miyaki, 1979), as well as no detectable surface associated lactobacilli (Tannock and Savage, 1974; Conway et al, 1986). The role the diet can play on colonization by lactobacilli is further empasised by the work of Lhuillery et al (1981). These workers showed that, while germ free mice fed either a commercial diet or a semi-synthetic diet were similarly colonized with a Lactobacillus strain, the off-spring of the semi-synthetic diet fed animals lacked the strain during lactation. The off-spring from the mice receiving commercial diet were rapidly colonized.

### REPORTED BENEFICIAL EFFECTS

The proposed beneficial effects range from direct bacterial interactions with pathogens to interactions with the host physiology and antitumour activities (Table 1). A general phenomenon throughout all roles is that the growth and most probably metabolites of lactobacilli exert an effect within the gut ecosystem. This effect may directly or indirectly be beneficial. The antimicrobial, anticholesteremic and anticarcinogenic activities have been recently reviewed (Fernandes et al, 1987), as has the enhanced bioavailability of nutrients and tolerance to lactose (Gurr, 1987). These can be broadly divided into antimicrobial, physiological and biochemical effects.

### Antimicrobial activitets

It has been shown that dosage of lactobacilli significantly decreases numbers of E. coli in vivo in chickens (Fuller, 1978), pigs (Barrow et al, 1980) and humans (Lidbeck et al, 1987), to cite just a few examples. Such an effect could lead to improved weight gain, improved egg production and quality, and decreased incidence of diarrhea and mortality but demonstration of such indirect effects has often been inconclusive. The lactobacilli have been shown in vitro to act directly on pathogens by inhibition of growth. This growth inhibition has been considered the major antagonistic action of lactobacilli. This view may be too simplified and perhaps it is more appropriate to talk in terms of competitive colonization which includes growth and colonization inhibition.

Lactobacillus primary metabolites such as lactic and acetic acid and hydrogen perioxide have been shown to be inhibitory to the growth of pathogens (e.g. reviewed Klaenhammer, 1982). In addition, low molecular weight metabolites with antagonistic properties have been reported, with some being active against specific strains (e.g. Barefoot and Klaenhammer, 1983; McCormick and Savage, 1983) while other workers report compounds with broad spectrum in vitro antagonistic activities (Goldin and Gorbach, 1987; Axelsson et al, 1987).

Ten Brink et al (1987) raised the issue that many reported antimicrobial compounds of lactobacilli are primary metabolites and proposed a new screening procedure. Using this method, two strains were shown to produce a protein-like antimicrobial compound active against a limited number of Gram positive bacteria.

The concept of competitive inhibition implies that the lactobacilli may out-compete the pathogens by either occupying the binding sites on the epithelia or exhausting the nutrients. This concept has been used by Nurmi and Rantala (1973) who implanted a mixture of non-pathogenic bacteria in the chicken to prevent pathogens establishing and Duval-Ifalh et al (1982) by introducing non-pathogenic E. coli in babies to competitively exclude antibiotic resistant E. coli. Pathogenic bacterial strains, e.g. enteropathogenic E. coli, colonize the gastrointestinal tract e.g. by adhering to the intestinal mucosa using proteinaceous fimbrial appendages such as the K88, K99, CFA/I, CFA/II, 987p and F41. Competition for the epithelial cell surface has been shown in an in vitro study in which E. coli cells were unable to adhere to epithelial cells already colonized by Lactobacillus acidophilus (Table 3).

**Table 3**

| Adhesion of E. coli K88 and L. acidophilus to pig ileal cells. | | |
|---|---|---|
| Bacteria | Pre-treatment* | Bacteria Adhering per ileal cell |
| L. acidophilus | - | 165 |
| E. coli | - | 78 |
| E. coli | + | 0 |

| | | |
|---|---|---|
| *Ileal cells incubated with L. acidophilus prior to assay | | |

In Swedish Patent Application No. 8405587-0, especially the new description filed on September 25, 1986, there is described a method of enhancing the adhesion of e.g. lactobacillus and Streptococcus to the gastrointestinal tract of experimental animals. It was found that the specific binding of the organisms to epithelial cells of the host organism is mediated by a certain protein, the so called APP (adhesive promoting protein). This method is thus concerned with the concept of outconcurring pathogens in the gastrointestinal tract by administering beneficial bacilli, which adhere to the epithelial mucosa and colonize the gut.

The present invention is concerned with the problem of gastrointestinal disturbances which are induced by, for example, antibiotic dosage or dietary alterations, resulting in pathogenic bacterial infections, for example, travellers diarrhea in humans, outbreaks of gastrointestinal infections in piglets.

One object of the invention is to provide products for inhibiting the adhesion of pathogens to gastrointestinal epithelium of humans and animals.

Another object of the invention is to provide products for inhibiting the growth of pathogens in animals including humans.

A further object is to provide products for inhibiting the survival of pathogens in animals including humans.

Yet another object of the invention is to provide a method of producing products for inhibiting the adhesion of pathogens to gastrointestinal epithelial mucosa, or for inhibiting the growth and/or survival of pathogens in animals including humans.

A further object of the invention is to provide preparations containing, as an active ingredient, products for inhibiting the adhesion of pathogens to gastrointestinal epithelial mucosa, or inhibiting the growth and/or survival of pathogens in animals including humans.

Another object of the invention is to provide preparations containing viable strains of Lactobacillus preferably originating from the gastrointestinal tract of the host to which the preparations will be administered.

A further object of the invention is to provide methods of inhibiting the adhesion of pathogens to gastrointestinal epithelial mucosa, or inhibiting the growth and/or survival of pathogens in animals including humans.

We have recently demonstrated a protein-like metabolite with a molecular weight greater than 8, 000 and that is active against a series of E. coli pig pathogens. These protein-like compounds may fall into the category of bacteriocins as described by Tagg (1976), who defined them as plasmid encoded, protein molecules which have a narrow bacteriocidal range and function by attachment to specific cell receptors.

In a separate study, preliminary results have shown that the adhesion of an E. coli K88 strain to pig intestinal mucosa was inhibited by the high molecular weight metabolites of lactobacilli isolated from the pig but not of lactobacilli from the mouse digestive tract (Table 4). Subsequent studies suggest that this was not the growth inhibitory compound and the mechanism of inhibition of adhesion is being investigated. Lactobacillus metabolites may inhibit pathogen colonization of the mucosal surface which is a prerequisite for pathogenicity for many strains. Consequently, factors in addition to growth inhibitory activities should also be considered.

**Table 4**

| Adhesion of E. coli K88 to pig mucus containing the receptor in the presence of high molecular metabolites of lactobacilli strains from the digestive tract of mouse and pig. | | |
|---|---|---|
| Metabolite source | Host origin | Adhesion to mucus |
| Buffer | - | 100% |
| L. fermentum | Mouse | 95% |
| L. murinus | Pig | 36% |
| Lactobacillus sp. strain 152 | Pig | 48% |

The products according to the invention consist of high molecular weight proteinaceous metabolites found in the culture supernatant of strains of crispatus, L. fermentum or L. murinus preferably originating from the gastrointestinal tract of the host to which the products will be administered.

Both low and high molecular weight compounds are inhibitory to pathogens. However, products according to the invention are high molecular weight, proteinaceous metabolites produced by strains of Lactobacillus crispatus, Lactobacillus fermentum or Lactobacillus murinus, which metabolites have the following characteristics:
a) they have molecular weights greater than 8,000;
b) they are heat sensitive;
c) they are sensitive to acid buffers;
d) they bind to enteropathogenic E. coli strains when growth inhibiting and to the mucus when adhesion inhibiting;
e) they have enhanced activity in the presence of lactic acid;
f) the production of which is favoured by growth in complex media rather than in semi-defined medium;
g) the production of which is favoured in semi-anaerobic growth conditions;
h) the production of which is optimal in the late log phase and the stationary phase of growth of the lactobacilli;
i) they are released into the growth medium.

As is stated above, it is known that primary metabolites of Lactobacillus, especially L. acidofilus, is inhibitory to the growth of pathogens, but it is totally new that also high molecular weight products are effective in this aspect. Also it is very surprising that the metabolites, both low and high molecular weight compounds, also inhibit the adhesion and survival of pathogens.

An optimal method of producing the products according to the invention is also provided. In this method a strain of Lactobacillus crispatus, Lactobacillus fermentum or Lactobacillus murinus preferably originating from the gastrointestinal tract of the host to which the products will be administered is cultured in complex media under semi-anaerobic growth conditions, whereby the optimal production of metabolites occurs in the late log phase and in the stationary phase of the growth, and the metabolites are recovered from the growth medium into which they are released during the culturing.

The preparations according to the invention can either comprise the metabolites defined above or viable strains of the Lactobacillus that have the capacity of producing the defined metabolites. In any cases, the preparations may comprise pharmaceutically acceptable constituents. The administration of viable cells will allow production of metabolites in vivo, without colonization being achieved or required.

The preparations according to the invention are administered to animals including, for instance, humans, piglets and chicken. The dosage could well be estimated by the person skilled in the art of human or veterinary medicine.

### Strain selection for dietary adjuncts

Many of the early studies concentrated on the beneficial effect of ingesting lactobacilli fermented milk products and lactobacilli strains used in the food industry. More recently, attention has been directed towards specifically selecting lactobacilli strains of gastrointestinal origin and with desirable characteristics (e.g. reviewed by Klaenhammer, 1982). This concept has already been extended to using host specific lactobacillus for producing a fermented milk product (Cole and Fuller, 1984; Ratcliffe et al, 1986).

Our first step in developing a lactobacillus preparation that can induce a beneficial effect, has been to define the target that is an antagonistic action against pathogens, and then develop a rapid screen to ensure that the lactobacilli to be used has this characteristic. In addition, it is cnfirmed that the selected strain has the capacity to remain viable and active in the digestive tract. The survival of lactobacilli cells in human stomach acidity and during transit through the tract has been investigated both in vivo and in vitro (Robins-Browne and Levine, 1981; Lindwall and Fonden, 1984; Pettersson et al, 1983a, 1983b; Lindwall and Fonden, 1984; Conway et al, 1987) and our strains have been selected with enhanced survival capacity.

### Stability of desired characteristics

Many of the earlier preparations were not consistent with regards to the viability of the preparation, as experienced by Clements et al (1983). These workers used different batches of the same commercial bacterial preparation and obtained conflicting results in human study.

There have been numerous examples of instability of specific character traits. Once a strain with the desired characteristics is selected, the stability of viability and various characteristics should be investigated because there have been numerous examples of instability of specific character traits. Instability will obvously lead to inconsistencies in results e.g. failure to observe antagonistic effects. We have observed a loss of the capacity to produce a high molecular weight bacteriocin-like compound from some gastrointestinal isolates while other strains of ours exhibit extremely stable antagonistic properties.

Lactobacilli strains isolated from the porcine gastric epithelium, including strains 152 and 104, exhibit an antagonistic effect on the growth of pathogenic E. coli. Strain 104 was administered to piglets in a field study and the results look very favourable as to the beneficial role that strain 104 has within the gastrointestinal tract.

In a similar study the human strain L. fermentum KLD3 was studied.

Within the gastrointestinal tract, the interactions between lactobacilli and coliforms encompass more than just growth inhibition. We have also considered the effect of lactobacilli on the colonization potential of the coliforms.

### Antagonistic effects of Lactobacillus spp. on E. coli colonization potential

We have studied the effects of lactobacillus metabolites on the capacity of E. coli K88 to adhere to ileal mucosa because adhesion via the K88 fimbriae represents the first step in the infection process. A number of lactobacillus strains have been examined and it was noted that L. fermentum typical strains produced metabolites with molecular weights greater than 8,000 (because they do not pass through dialysis tubing with that size cut-off) which inhibited the adhesion of the K88 fimbriae. The mode of action appears to be that the lactobacilli metabolites bind to the mucus and thereby inhibit adhesion. Further fractionation of the metabolites showed that the active fraction has a molecular weight greater than 30,000 and that it is not the same compound which inhibits growth of the E. coli as presented below. The presence of the K88-adhesion-inhibitory substance was influenced by the nutrients supplied to the lactobacilli.

### Antagonistic effect of Lactobacillus crispatus strain 104 on growth of E. coli

Lactobacillus crispatus strain 104 was found to inhibit the growth of E. coli strain C23. Inhibition was not only found with the crude supernatant but also with dialysed supernatant, indicating a substance other than lactic acid, acetic acid or H₂O₂ as the antagonist. We have determined the optimal growth conditions for the production of this antagonistic product by strain 104, characterized the structure and function of the antagonist, and examined the range of bacteria which are sensitive to this antagonistic product.

Characterization of L. crispatus strain 104 revealed that it consisted of two different colony variants, smooth and rough, when plated on agar plates. On MRS-agar plates smooth colonies were exactly round and shiny, whereas the rough colonies were irregular in shape and dull.

To be able to find optimal conditions for the production of the antagonist by strain 104 rb, the influence of carbon source, oxygen tension, and growth phase were investigated. Glucose was the only carbon source, out of 10 tested, that allowed the production of the antagnist. Although strain 104 rb grew well on saccharose, melobiose, maltose, and raffinose, no antagonistic activity could be detected in the supernatant of these cultures. Semi-anaerobic conditions, as present in anaerobic jars with a lighted candle, favoured the production of the antagonist. In the supernatant of cultures, uncubated unshaken under aerobic conditions less activity was found, and no activity at all in the supernatant of anaerobic cultures. Strain 104 rb did not grow in aerated (shaken) flasks. Test for antagonistic activity of supernatants from different growth phases of strain rb showed that most of the antagonist was produced in the stationary phase.

Parallel to these experiments the antagonistic product of strain 104 rb was characterized. From the cut off of the dialysis membrane it was known that the molecular weight was higher than 8000. In further experiments it could be shown that the antagonistic product was heat sensitive and sensitive to acid buffers. When the dialysed supernatant of strain 104 rb was pre-incubated with E. coli, recentrifuged to remove the E. coli cells and then tested, no growth inhibition of E. coli was found. This indicated that the antagonist was binding to E. coli cells. All these characteristics make it plausible that the antagonistic product of strain 104 rb is a protein.

In all the experiments mentioned above the antagonistic effect was measured as growth inhibition of E. coli. E. coli was inoculated in medium containg the dialysed supernatant of strain 104 rb. By recording the growth of E. coli in comparison to a control, containing dialysed medium instead of supernatant, the inhibition of E. coli could be recorded. Since the antagonistic effect was only small in these experiments, investigations were carried out, how the sensitivity of E. coli against the antagonist could be increased. E. coli was much more sensitive when the supernatant of strain 104 rb was added to stationary phase cells, together with the amount of lactic acid, acetic acid, and ethanol produced by strain 104 rb in BHI containing 2% glucose (Tab. 5). The antagonist significantly increased the effect of the acids and ethanol on E. coli. While 6.3 x 10⁵ CFU of E. coli were found in the control after 12 hours of incubation, only 2.4 x 10⁴ CFU were found in the cultures with added supernatant. The same results were obtained, when E. coli was cultivated for 12 hours in medium with 2% glucose before the supernatant of strain 104 rb was added (Tab. 6).

**Table 5**

| Cell number of E. coli C 23 after 24 hours of incubation. Cultures were grown in BHI medium for 12 hours (stationary phase) before additions were made. | |
|---|---|
| Addition | Cell number after 24 h |
| Control | 4.2 x 10⁸ |
| Supernatant | 4.6 x 10⁸ |
| Control + lactic acid + ethanol + acetic acid | 6.3 x 10⁵ |
| Supernatant + lactic acid + ethanol + acetic acid | 2.4 x 10⁴ |

**Table 6**

| Cell number of E. coli C 23 after 24 hours of incubation. Cultures were grown in BHI medium with 2% glucose for 12 hours (stationary phase) before additions were made. | |
|---|---|
| Addition | Cell number after 24 h |
| Control | 3.3 x 10⁷ |
| Supernatant | 1.1 x 10⁵ |

### Effect of Lactobacillus strain 104 on the growth of E. coli K88

E. coli K88 was grown under anaerobic conditions in BHI medium (brain heart infusion, Oxoid) with 2% glucose in the presence and in the absence of Lactobacillus strain 104r or 104s.

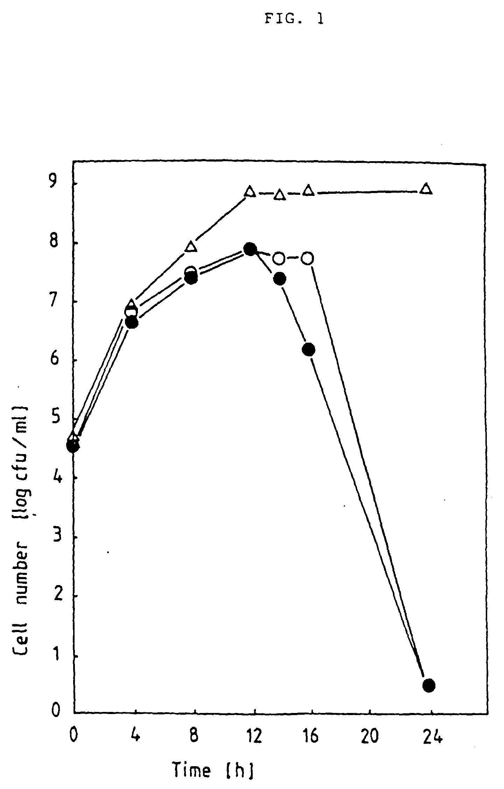

In the cultures were Lactobacillus strain 104r or 104s were present, the growth rates of E. coli were reduced and the viability of E. coli K88 in the stationary phase dramatically decreased (Fig. 1). While 6.3 x 10⁸ cfu of E. coli K88 were found in the control culture after 24 h, less than 10 viable cells were present in the cultures with the lactobacilli.

To exclude effects of low molecular weight acids and H₂O₂ produced by the lactobacilli, the supernatant was dialysed and then tested for effects on growth (B.) and viability (C.) of E. coli K88.

The results are shown in fig. 1.

### Effect of dialysed supernatant on the growth of E. coli K88

Dialysed supernatants of 28 h cultures of Lactobacillus train 104r or 104s, BHI + 2% glucose grown, were mixed 1:1 with anoxic BHI medium and inoculated with E. coli K88. Dialysed BHI medium was used as control. The pH was adjusted to 6.0 in all experiments.

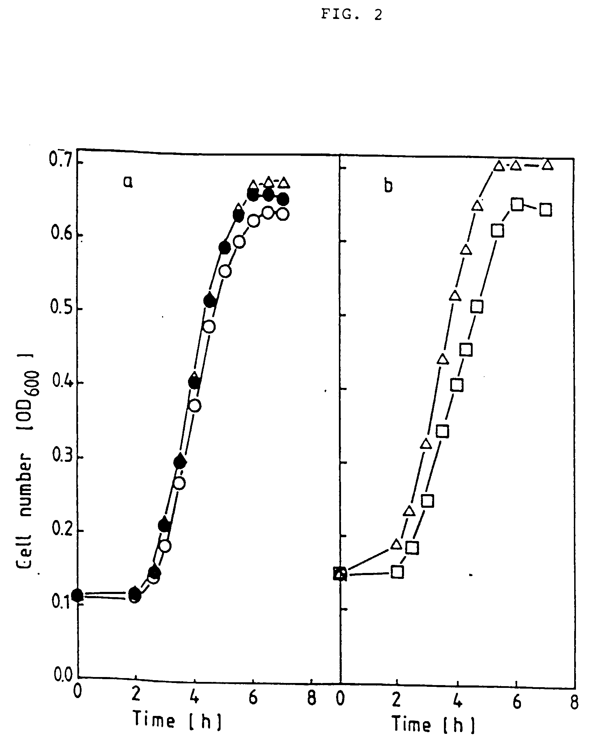

The growth of E. coli K88 was only slightly affected by supernatant of the smooth variant of strain 104, while the rough variant supernatant clearly reduced growth (Fig. 2a). The growth inhibition was enhanced 4-fold when the supernatant of strain 104r was concentrated 10-fold by freeze drying (Fig. 2b).

The results are shown in fig. 2.

### Effect of the dialysed supernatant on the viability of E. coli K88

E. coli K88 was pregrown to stationary phase in BHI with 2% glucose, before the addition of supernatant of Lactobacillus strain 104r or 104s. Dialysed BHI served as control supernatant. The pH was adjusted to 5.0 in all experiments. After 24 h, the number of viable E. coli K88 cells was determined by plate counts.

About 1000 times less viable cells were found in the cultures were the supernatants of the lactobacilli were added as compared to the control (Table 6). The culture supernatant of the smooth variant had a stronger inhibitory effect than the rough variant.

**Table 7**

| Cell numbers of E. coli K88, 24 h after the addition of the lactobacilli supernatants or dialysed BHI. Values given are means of three independent measurements. | |
|---|---|
| Addition | Cell number |
| dialysed BHI | 1.9 x 10⁷ |
| supernatant of strain 104r | 2.1 x 10⁴ |
| supernatant of strain 104s | 1,0 x 10⁴ |

### Characteristics of a Lactobacillus spp antagonistic effect against pathogenic Escherichia coli

The metabolites of Lactobacillus strain 104 of porcine gastrointestinal origin were examined for their inhibitory effect on the growth of a pathogenic E. coli K88 strain. Preliminary studies showed that the growth of E. coli strain was inhibited not only by the crude culture supernatant but also after dialysis of the spent culture fluid, indicative of the presence of active components other than lactic acid and acetic acid or hydrogen peroxide. The culture of Lactobacillus strain 104 on agar plates yielded two different colony variants, smooth and rough. The rough strains showed an at least 10 times enhanced antagonistic activity against growth of E. coli K88 than the smooth strains. In addition the rough and smooth strains showed different adhesive capacities. For the rough strain 104 rb, the stability in MRS, BHI or LDM broth was examined and the antagonistic metabolite characterized. In addition, the influence of the carbon source, oxygen tension, and growth phase on the activity of the antagonist were investigated. It was found that E. coli growth was inhibited by a high molecular weight compound that was heat and acid sensitive. The antagonist bound to E. coli cells and from the data it is plausible that this metabolite of strain 104 rb is proteinaceous.

### Inhibition of adhesion of enterotoxigenic Escherichia coli K88 to pig ileal mucus by metabolites of Lactobacillus crispatus strain 104

The effect of metabolites of L. crispatus on adhesion of E. coli K88 to piglet ileal mucus was investigated. This was studied in an in vitro adhesion assay by pretreating immobilized mucus with Lactobacillus metabolites. These were prepared from overnight culture supernatants of L. crispatus by dialyzing and then fractionating through ultra filters with the cut off at the molecular weight of 10,000 and 30,000. It appears that there are metabolites produced when L. crispatus is grown in BHI or LDM broth, that adhere to the ileal mucus and thereby inhibit adhesion of E. coli K88. The active fraction, which is partially destroyed by heating, has a molecular weight greater than 30,000. Treatment with periodate, however, reduced the antagonistic effect, suggesting that compound(s) with carbohydrate constituents are active in the blocking of the receptor for the K88 fimbriae.

### Optimal culture conditions for the production of the antagonistic product by Lactobacillus strain 104 Stability with subculturing

The antagonistic action of the supernatant against E. coli K88 remained unchanged after 100 transfers of the cultures in liquid BHI or MRS medium or repeated freezing of the cultures.

### Medium composition

Complex media, such as BHI or MRS, favoured the production of the antagonistic product.

Production was reduced in dialysed BHI medium or LDM medium (semi-defined). Glucose was the only carbon source, out of 10 tested in LDM medium, that allowed the production of the antagonist.

### Oxygen tension

Semi-anaerobic growth conditions favoured the production of the antagonist. Less activity was found in the supernatant of cultures, incubated unshaken under aerobic conditions.

Nearly no activity was found in the supernatant of anaerobic cultures.

### Growth phase

Most of the antagonist was produced in the stationary phase.

### Characterization of the antagonistic product

1. Antagonistic effect not due to:
   - bacteriophage
      Dilution to extinction of the supernatant of the lactobacilli cultures gave no bacteriophage plaques.
   - hydrogen peroxide or low molecular weight acids
      The antagonistic effect was still present in dialysed supernatant.
   - low pH
      The pH in the controls and the cultures with added supernatant was adjusted to the same value.
2. Properties of the antagonistic product
   - > 10000 Dalton
      The dialysed supernatant still contained the antagonistic activity.
   - heat sensitive
      Heating of the supernatant to 100,5°C for two minutes destroyed the antagonist.
   - sensitive to acid buffers
      After dialysing the supernatant against acid buffers no antagonistic activity was detected.
   - not sensitive to pronase or trypsin
      Addition of pronase or trypsin had no effect on the action of the antagonist against E. coli K88.
   - binds to E. coli K88
      When the supernatant of Lactobacillus strain 104r was pre-incubated with E. coli K88, re-centrifuged to remove the E. coli cells and then tested, no growth inhibition of E. coli was found.
      These features make it plausible that the antagonist is a protein which is not sensitive to pronase and trypsin.

### Antagonistic effects of Lactobacillus fermentum of human origin on pathogens

The results using method 101/1 are presented in Table 8 and from the data it can be consluded that L. fermentum strain KLD has a marked antagonistic action on all bacterial isolates tested.

**Table 8**

| Antagonistic action of L. fermentum KLD against a range of strains. All strains are human pathogens except E. coli Sb5 and Streptococcus faecium which are indigenous to the mouse gastrointestinal tract. Results are expressed as the size of the zone inhibition of growth of the pathogen when overlaid on colonies of L. fermentum KLD after growth of the KLD strain in MRS broth. | |
|---|---|
| Bacterial strain | Zone of inhibition (mm) |
| Campylobacter jejuni | 12 |
| E. coli C21 | 7 |
| E. coli C22 | 7 |
| E. coli H10407 | 7 |
| E. coli 334 | 8 |
| E. coli B2C | 7 |
| E. coli -R | 7 |
| E. coli KS219 | 7 |
| E. coli Sb5 | 6 |
| Salmonella sofia | 7 |
| " eq.ph | 7 |
| Streptococcus faecium | 6 |
| Method 101/1 Screening for antagonistic metabolites | |

This screening method can be used for either actively growing lactobacillus cultures or for bacterial suspensions prepared from the freeze dried preparations. The latter suspensions were prepared by shaking 2 g of dried powder together with 18 ml PBS. A point inoculum (10 µl) of the overnight culture, or the bacterial suspension in PBS, was placed on an BHI + 2% glucose plate. Two such point inocula were tested per plate. After 48 h incubation at 37°C in a candle jar, the pathogen to be tested was overlaid on top of the lactobacillus colony. This overlay consisted of 10 µl of an overnight culture of the pathogen in 3 ml soft agar. The plates were incubated overnight and the growth inhibition measured by the size of the zone of no growth of the pathogen around the lactobacillus colony. To compare from one plate and analysis to another, consistently uniform plates were prepared.

### REFERENCES

Adams, R.F. (1980). Some effects of nutrients, non-nutrients and food poisoning organisms on the intestinal microbial system. Food Technol. Aust. 30,92-98. Adams, R.F. and Conway, P.L. (1981). The effect of erythrosine on the surface-associated bacteria of the rat stomach and caecum. J. Appl. Bacteriol. 51,171-181. Alm, L. (1980). Acidophilusmjölk och dess terapeutiska värde. Näringsforskning 3/80,91-95.

Alm, L., Humble, D., Ryd-Kjellen, E. and Setterberg, G. (1983). The effect of acidophilus milk in the treatment of constipation in hospitalized geriatric patients. Nutrition and the intestinal flora. Proc. Symposia of the Swedish nutrition foundation 15. (Hallgren, B. ed.) Almqvist and Wiksell, pp 131-138.

Axelsson, L., Chung, T.C., Dobrogosz, W.J. and Lindgren, L.E. (1987). Discovery of a new antimicrobial substance produced by Lactobacillus reuteri. FEMS Microbiol. Rev. 46,P65.

Barefoot, S.F. and Klaenhammer, T.R. (1983). Detection and activity of lactacin B, a bacteriocin produced by Lactobacillus acidophilus. Appl. Environ. Microbiol. 45,1808-1815.

Barrow, P.A., Brooker, B.E., Fuller, R. and Newport, M.J. (1980). The attachment of bacteria to the gastric epithelium of the pig and its importance in the microecology of the intestine. J. Appl. Bacteriol. 48,147-154.

Belonovsky, J. (1907). Influence du ferment lactique sur la flore des excréments des souris. Ann. Inst. Pasteur, Paris. 21,991-1004.

Bettelheim, K.A., Teoh-Chan, C.H., Chandler, M.E., O'Farrell, S.M., Rahamin, L., Shaw, E.J. and Shooter, R.A. (1974). Further studies of Escherichia coli in babies after normal delivery. J. Hyg. 73,277-285.

Brockett, M. and Tannock, G.W. (1981). Dietary cmponents influence tissue-associated lactobacilli in the mouse stomach. Can. J. Microbiol. 27,452-455.

Brownlee, A. and Moss, W. (1961). The influence of diet on lactobacilli in the stomach of the rat. J. Path. Bact. 82,513-531.

Cohendy, M. (1906a). Essais d'acclimation microbienne dans la cavite intestinale. C. R. Soc. Biol. Paris, 60, 364-366.

Cohendy, M. (1906). Description du ferment lactique puissant de s'acchimater dans l'entestine de l'homme. C. R. Soc. Biol. Oaris, 60, 558-561.

Cole, C.B. and Fuller, R. (1984). A note on the effect of host specific fermented milk on the coliform population of the neonatal rat gut. J. Appl. Bacteriol. 56,495-498. Conway, P.L., Maki, J., Mitchell, R. and Kjellerberg, S. (1986). Starvation of marine flounder, squid and laboratory mice and its effect on the intestinal microbiota. FEMS Microbiol. Ecol. 38,187-195.

Conway, P.L., Gorbach, S.L., and Goldin, B.R. (1987). Survival of lactic acid bacteria in the human stomach and adhesion to intestinal cells. J. Dairy Sci. 70,1-12. Clements, M.L., Levine, M.M., Ristaino, P.A., Daya, V.E. and Hughes, T.P. (1983). Exogenous lactobacilli fed to man - their fate and ability to prevent diarrheal disease. Prog. Fd. Nutr. Sci. 7,29-37.

Distaso, A. and Schiller, J. (1914). Sur l'acclimatation dans le gros intestin de microbes étrangers à la flore intestinale. C. R. Soc. Biol. Paris. 76,243-244. Dubos, R.J. and Schaedler, R.W. (1962). The effect of diet on the fecal bacterial flora of mice and on their resistance to infection. J. Exp. Med. 115,1161-1171. Ducluzeau, R. (1983). Implantation and development of the gut flora in the newborn animal. Ann. Rech. Vet. 14,354-359.

Duval-Iflah, Y., Ouriet, M.F., Moreau, C., Daniel, J.C., Gabilan, J.C. and Raibaud, P. (1982). Implantation precoce d'une souche de Escherichia coli dans l'intestin de noveau-nes humains: effect de barriere vis-a-vis de souches de E. coli antibioresitantes. Ann. Microbiol. 133A,393-408.

Edmiston, C.E., Jr., Avant, G.R. and Wilson, F.A. (1982). Anaerobic bacterial populations on normal and diseased human biopsy tissue obtained at colonoscopy. Appl. Environ Microbiol. 43,1173-1181.

Evaldson, G., Heimdahl, A., Kager, L. and Nord C.E. (1982). The normal human anaerobic microflora. Scand. J. Infect. Dis. Suppl. 35,9-15.

Fernandes, C.F., Shanhani, K.M. and Amer, M.A. (1987). Therapeutic role of dietary lactobacilli and lactobacilli fermented dairy products. FEMS Microbiol. Rev. 46,343-356. Freter, R. (1955). The fatal enteric cholera infection in the guinea pig, achieved by inhibition of normal enteric flora. J. Infect. Dis. 97,57-65.

Freter, R. (1956). Experimental enteric Shigella and Vibrio infections in mice and guinea pigs. J. Exp. Med. 104, 411-418.

Fuller, R. (1973). Ecological studies on the lactobacillus flora associated with the crop epithelium of the fowl. J. Appl. Bacteriol. 36,131-139.

Fuller, R. (1978). Epithelial attachment and other factors controlling the colonization of the intestine of the gnotobiotic chicken by lactobacilli. J. Appl. Bacteriol. 45,389-395.

Fuller, R. (1986). Probiotics. J. Appl. Bacteriol. Symp. Suppl. 1S-7S.

Fuller, R. and Turvey, A. (1971). Bacteria associated with the intestinal wall of the fowl Gallus domesticus. J. Appl. Bacteriol. 34,617-622.

Gilliland, S.E., Nelson, C.R. and Maxwell, C. (1985). Assimilation of cholesterol by Lactobacillus acidophilus. Appl. Environ. Micro. 49,377-381.

Gilliland, S.E. and Speck, M.L. (1977). Deconjugation of bile acids by intestinal lactobacilli. Appl. Environ. Microbiol. 33,15-18.

Goldin, B.R. and Gorbach, S.L. (1980). Effect of Lactobacillus acidophilus dietary supplements on 1,2-dimethylhydrazine dihydrochloride induced intestinal cancer in rats. J. Natl. Cancer Inst. 64,263-271.

Goldin, B.R. and Gorbach, S.L. (1987). Lactobacillus GG: a new strain with properties favorable for survival, adhesion and antimicrobial activity in the gastrointestinal tract. FEMS Microbiol. Rev. 46,P72.

Goldin, B.R., Swensson, L., Dwyer, J., Sexton, M. and Gorbach, S.L. (1980). Effect of diet and L. acidophilus supplements on human fecal bacterial enzymes. J. Natl. Cancer Inst. 64,255-262.

Grunewald, K.K. (1982). Serum cholesterol levels in rats fed skim milk fermented by Lactobacillus acidophilus. J. Food Sci. 47,2078-2079.

Gurr, M. (1987). Nutritional aspects of fermented milk products. FEMS Microbiol. Rev. 46,337-342.

Hentges D.J. (1983). Role of the intestinal microflora in host defense against infection. In: "Human intestinal microflora in health and disease" (ed. Hentges, D.J.), Academic Press, London, pp 311-331.

Herter, C.A. and Kendall, A.I. (1908). An observation on the fate of B. bulgaricus in the digestive tract of a monkey. J. Biol. Chem. 5,293-302.

Hull, T.G. and Rettger, L.F. (1914). The influence of milk and carbohydrate feeding on the intestinal flora of white rats. Zentralbl. Bacteriol. Abt. I, Orig. A 75,219-229.

Jonsson, E. (1986). Persistence of Lactobacillus strain in the gut of suckling piglets and its influence on performance and health. Swedish J. agric. Res. 16,43-47.

Klaenhammer, T.R. (1982). Microbiological considerations in selection and preparation of lactobacillus strains in use as dietary adjuncts. J. Dairy Sci. 65,1339-1349.

Kleeman, E.G. and Klaenhammer, T.R. (1982). Adherence of lactobacillus sp to human fetal intestinal cells. J. Dairy Sci. 65,2063-69.

Kolars, J.C., Levitt, M.D., Aouji, M. and Savaiano, D.A. (1984). Yogurt: and autodigesting source of lactose. N. Engl. J. Med. 310,1-3.

Lessard, M. and Brisson, G.J. (1987). Effect of lactobacillus fermentation product on growth immune response and fecal enzyme activity in weaned pigs. Can. J. Anim. Sci. 62,509-516.

Lhuillery, C., Demarne, Y., Dubos, F., Galpin, J.-V., Ducluzeau, R. and Raibaud, P. (1981). Fatty acid composition of lipids in the maternal diet and establishment of a Lactobacillus sp. strain in the digestive tract of suckling gnotobiotic mice and rats. Am. J. Clin. Nutr. 34,1513-1519.

Lidbeck, A., Gustafsson, J-A. and Nord, C.E. (1987). Impact of Lactobacillus acidophilus supplements on the human oropharyngeal and intestinal microflora. Scand. J. Infect. Dis. 19,531-537.

Lindwall, S. and Fonden, R. (1984). Passage and survival of L. acidophilus in the human gastrointestinal tract. Int. Dairy Fed. Bull. FIL 179. XXI.

Lizko, N.N., Silov, V.M. and Syrych, G.D. (1984). Die Besonderheiten der Bildung einer Dysbakteriose des Darms beim Menschen unter Extrembdedingungen. Die Nahrung 28,599-605.

Luckey, T.D. (1984). Perspectives in intestinal ecology. Microecol. Ther. 14,243-249.

Luerssen, A. and Kühn, M. (1908). Yoghurt, die bulgarische Sauermilch. Zentralbl. Bakteriol. 20,234-248.

Petterson, L., Graf, W. and Sewelin, V. (1983b). Survival of Lactobacillus acidophilus NCDO 1748 in the human gastrointestinal tract. 2. Ability to pass the stomach and intestine in vivo. Nutrition and the intestinal flora. in Symp. Swed. Nutr. Found. 15. Bo Hallgren, ed. Uppsala. pp 127.

Plaut, A.G., Gorbach, S.L., Nahas, L. and Weinstein, L. (1967). Studies of intestinal microflora. III. The microbial flora of human small intestinal mucosa and fluids. Gastroenterology 53,868-873.

Pollmann, D.S., Danielson, D.M., Wren, W.B., Peo, Jr., E.R. and Shahani, K.M. (1980). Influence of Lactobacillus acidophilus inoculum on gnotobiotic and conventional pigs. J. Animal Sci. 51,629-637.

Porter, J.R. and Rettger, L.F. (1940). Influence of diet on the distribution of bacteria in the stomach, small intestine and cecum of the white rat. J. Infect. Dis. 6,104-110.

Pradham, A. and Majumdar, M.K. (1986). Metabolism of some drugs by intestinal lactobacilli and their toxilogical considersations. Acta pharmacol. et. toxicol. 58,11-15.

Ratcliffe, B., Cole, C.C., Fuller, R. and Newport, M.J. (1986). The effect of yoghurt and milk fermented with a porcine intestinal strain of Lactobacillus reuteri on the performance and gastro intestinal flora of pigs weaned at two days of age. Food. Microbiol. 3,203-211.

Robins-Browne, R.M. and Levine, M.M. (1981). The fate of ingested lactobacilli in the proximal small intestine. Am. J. Clin. Nutr. 34,514-519.

Robinson, E.L. and Thompson, W.L. (1952). Effect on weight gain of the addition of Lactophilus acidophilus to formula of newborn infants. J. Pediat. 41,395-398.

Sandine, W.E. (1979). Roles of lactobacillus in the gastrointestinal tract. J. Food Protect. 42,259-262. Sandine, W.E., Muralidhara, K.S., Elliker, P.R. and England, D.C. (1972). Lactic acid bacteria in food and health: A review with special reference to enteropathogenic Escherichia coli as well as certain enteric diseases and their treatment with antibiotics and lactobacilli. J. Milk Food Technol. 35,691-702.

Savage, D. (1977). Microbial ecology of the gastrointestinal tract. Annu. Rev. Microbiol. 31,107-133.

Shahani, K. and Ayebo, A.D. (1980). Role of dietary lactobacilli in gastrointestinal microecology. Amer. J. Clin. Nutr. 33,2448-2457.

McCormick, E.L. and Savage, D.C. (1983). Characterization of Lactobacillus sp. strain 100-37 from the murine gastrointestinal tract: ecology, plasmid content, and antagonistic activity toward Clostridium ramosum H1. Appl. Environ. Microbiol. 46,1103-1186.

Miles, R.D., Arafa, A.S., Harms, R.H., Carlsson, C.W. Reid, B.L. and Crawford, J.S. (1981). Effects of a living nonfreeze-dried Lactobacillus acidophilus culture on performance, egg quality, and gut microflora in commercial layers. Poult. Sci. 60,993-1004.

Morishitya, Y. and Miyaki, K. (1979). Effects of aging and starvation on the gastrointestinal microflora and the heat resistance of faecal bacteria in rats. Microbiol. Immunol. 23,455-470.

Morotomi, M., Watanabe, T., Suegara, N., Kawai, Y. and Mutai, M. (1975). Distribution of indigenous bacteria in the digestive tract of both conventional and gnotobiotic rats. Infect. Immun. 1,962-968.

Muralidhara, K.S., Sheggeby, G.G., Elliker, P.R., England, D.C. and Sandine, W.E. (1977). Effect of feeding lactobacilli on the coli form and Lactobacillus flora of intestinal tissue and feces from piglets. J. Food Protect. 40,288-296.

Nisse, A. (1916). Ueber die Grundlagen einer ursaechlichen Bekaempfung der pathologischen Darmflora. Dtsch. Med. Wochenschr. 42,1181-1184.

Nelson, D.P. and Mata, L.J. (1970). Bacterial flora associated with human gastrointestinal mucosa. Gastroenterology 58,56-61.

Nurmi, E. and Rantala, M. (1973). New prospects of Salmonella infection in broiler production. Nature 241,210-211. Pedersen, K. and Tannock, G. (1986). Colonization and persistence of two lactobacillus strains in the digestive tract of piglets. FEMS Microbiol. Rev. 46,P74.

Perdigon, G., Nader de Macias, M.E., Alvarez, S., Oliver, G. and Pesce de Ruiz Holgado, A.A. (1987). Enhancement of immune response in mice fed with Streptococcus thermophilus and Lactobacillus acidophilus. 70,919-926.

Petterson, L., Graf, W., Alm, L. Lindwall, S. and Strindberg, A. (1983a). Survival of Lactobacillus acidophilus NCDO 1748 in the human gastrointestinal tract. 1. Incubation in vitro. Nutrition and the intestinal flora. In Symp. Swed. Nutr. Found. 15. Bo Hallgren, ed. Uppsala. pp 123.

Speck, M.L. (1976). Interaction among lactobacilli and man. J. Dairy Sci. 59,338-343.

Tannock, G.W. (1983). Effect of dietary and environmental stress on the gastrointestinal microbiota. In: "Human Intestinal Microflora in Health and Disease" (Hentges, D. ed.) Academic Press, London, pp 517-539.

Tannock, G.W. and Savage, D.C. (1974). Influence of dietary and environmental stress on microbial populations in the murine gastrpointestinal tract. Infect. Immun. 9,591-598.

Tannock, G.W. and Smith, J.M.B. (1970). The microflora of the pig stomach and its possible relationship to ulceration of the pars oesophagea. J. Comp. Path. 80,359-367. Ten Brink, B., Minekus, M., Bol, J. and Huis, in 't Veld, J.H.J. (1987). Production of antimicrobial compounds by lactobacilli. FEMS Microbiol. Rev. 46,P64.

Tuschy, D. (1986). Verwendung von "probiotika" als leistungsförderer in der tiernährung. Obers. Tiernährg. 14,157-178.

Tzipori, S., McCartney, E., Chang, H.S. and Dunkin, A. (1984). Postweaning diarrhoea in pigs: an interaction between change to dry food and colibacillosis. FEMS Microbiol. Letters 24,313-317.

Willis, A.T., Bullen, C.L., Williams, K., Fag, C.G., Bourne, A. and Vignon, M. (1973). Breast milk substitute: A bacteriological study. Br. Med. J. 4,67-72.

Yoshioka, H., Iseiki, K. and Fujita, K. (1983). Development and differences of intestinal flora in the neonatal period in breast-fed and bottle-fed infants. Pediatrics 72,317-321.

### FIGURE LEGENDS

- FIG 1:: Viable cell number of E. coli K88 during growth in BHI + glucose with or without addition of lactobacilli.
Δ E. coli K88 alone
o E. coli K88 and Lactobacillus strain 104r
● E. coli K88 and Lactobacillus strain 104s
- FIG 2:: Growth of E. coli K88 in BHI (Δ) with addition of
a) supernatant of Lactobacillus strain 104r (o)
   supernatant of Lactobacillus strain 104s (●)
b) 10-fold concentrated supernatant of strain 104r (□)

## Claims

1. Products for inhibiting the adhesion of pathogens to gastrointestinal epithelial mucosa, or for inhibiting the growth and/or survival of pathogens in animals including humans, **characterised** in that they consist of high molecular weight, proteinaceous metabolites found in the culture supernatant of strains of Lactobacillus crispatus, Lactobacillus fermentum or Lactobacillus murinus, preferably originating from the gastrointestinal tract of the host to which the products will be administered, which metabolites have the following characteristics:
a) they have molecular weights greater than 8,000;
b) they are heat sensitive;
c) they are sensitive to acid buffers;
d) they bind to enteropathogenic E. coli strains when growth inhibiting and to the mucus when adhesion inhibiting;
e) they have enhanced activity in the presence of lactic acid;
f) the production of which is favoured by growth in complex media rather than in semi-defined medium;
g) the production of which is favoured under semi-anaerobic growth conditions;
h) the production of which is optimal in the late log phase and the stationary phase of growth of the lactobacilli;
i) they are released into the growth medium.

2. A method of producing products for inhibiting the adhesion of pathogens to gastrointestinal epithelial mucosa, or for inhibiting the growth and/or survival of pathogens in animals including humans, which products are high molecular weight, proteinaceous metabolites produced by strains of Lactobacillus crispatus, Lactobacillus fermentum or Lactobacillus murinus, preferably originating from the gastrointestinal tract of the host to which the products will be administrered, which metabolites have the following characteristics:
a) they have molecular weights greater than 8,000;
b) they are heat sensitive;
c) they are sensitive to acid buffers;
d) they bind to enteropathogenic E. coli strains when growth inhibiting and to the mucus when adhesion inhibiting;
e) they have enhanced activity in the presence of lactic acid;
f) the production of which is favoured by growth in complex media rather than in semi-defined medium;
g) the production of which is favoured under semi-anaerobic growth conditions;
h) the production of which is optimal in the late log phase and the stationary phase of growth of the lactobacilli;
i) they are released into the growth medium,
**characterised** in that a strain strain of Lactobacillus preferably originating from the gastrointestinal tract of the host to which the products will be administrered is cultured in complex media under semi-anaerob growth conditions, whereby the optimal production of metabolites occurs in the late log phase and in the stationary phase of the growth, and the metabolites are recovered from the growth medium into which thery are released during the culturing.

3. Preparations containing, as an active ingredient, high molecular weight, proteinaceous metabolites found in the culture supernatant of strains of Lactobacillus crispatus, Lactobacillus fermentum or Lactobacillus murinus, preferably originating from the gastrointestinal tract of the animals to which the preparations will be administrered, and, optionally, pharmaceutically acceptable constituents, which metabolites have the following characteristics:
a) they have molecular weights greater than 8,000;
b) they are heat sensitive;
c) they are sensitive to acid buffers;
d) they bind to enteropathogenic E. coli strains when growth inhibiting and to the mucus when adhesion inhibiting;
e) they have enhanced activity in the presence of lactic acid;
f) the production of which is favoured by growth in complex media rather than in semi-defined medium;
g) the production of which is favoured under semi-anaerobic growth conditions;
h) the production of which is optimal in the late log phase and the stationary phase of growth of the lactobacilli;
i) they are released into the growth medium.

## Patentansprüche

1. Produkte zur Inhibierung der Haftung von Pathogenen an der gastrointestinalen Epithelschleimhaut oder zur Inhibierung des Wachstums und/oder Überlebens von Pathogenen in Tieren einschließlich Menschen, dadurch gekennzeichnet, daß sie aus hochmolekulargewichtigen, proteinhaltigen Metaboliten bestehen, die im Kultur-Überstand von Stämmen des Lactobacillus crispatus, Lactobacillus fermentum oder Lactobacillus murinus gefunden werden, vorzugsweise abstammend vom Gastrointestinaltrakt des Wirtes, an welchen die Produkte verabreicht werden, welche Metaboliten die folgenden Charakteristika aufweisen:
a) sie haben Molekulargewichte von mehr als 8.000;
b) sie sind wärmeempfindlich;
c) sie sind gegenüber Säurepuffern empfindlich;
d) sie binden an enteropathogene E. coli-Stämme, wenn sie wachstumsinhibierend sind, und an den Mucus, wenn sie haftungsinhibierend sind;
e) sie haben in Gegenwart von Milchsäure verstärkte Aktivität;
f) ihre Produktion wird begünstigt durch Wachstum in Kornplexmedien, im Gegensatz zu halbdefiniertem Medium;
g) ihre Produktion ist begünstigt unter semianaeroben Wachstumsbedingungen;
h) ihre Produktion ist optimal in der späten log-Phase und der stationären Wachstumsphase der Lactobacilli;
i) sie werden in das Wachstumsmedium freigesetzt.

2. Verfahren zur Herstellung von Produkten zur Inhibierung der Haftung von Pathogenen an gastrointestinaler Epithelschleimhaut oder zur Inhibierung des Wachstums und/oder Überlebens von Pathogenen in Tieren einschließlich Menschen, welche Produkte hochmolekulargewichtige, proteinhaltige Metabolite sind, erzeugt durch Stämme des Lactobacillus crispatus, Lactobacillus fermentum oder Lactobacillus murinus, vorzugsweise abstammend vom Gastrointestinaltrakt des Wirtes, an welche die Produkte verabreicht werden, welche Metaboliten die folgenden Charakteristika aufweisen:
a) sie haben Molekulargewichte von mehr als 8.000;
b) sie sind wärmeempfindlich;
c) sie sind gegenüber Säurepuffern empfindlich;
d) sie binden an enteropathogene E. coli-Stämme, wenn sie wachstumsinhibierend sind, und an den Mucus, wenn sie haftungsinhibierend sind;
e) sie haben in Gegenwart von Milchsäure verstärkte Aktivität;
f) ihre Produktion wird begünstigt durch Wachstum in Komplexmedien, im Gegensatz zu halbdefiniertem Medium;
g) ihre Produktion ist begünstigt unter semianaeroben Wachstumsbedingungen;
h) ihre Produktion ist optimal in der späten log-Phase und der stationären Wachstumsphase der Lactobacilli;
i) sie werden in das Wachstumsmedium freigesetzt;
dadurch gekennzeichnet, daß ein Lactobacillus-Stamm, der vorzugsweise vom Gastrointestinaltrakt des Wirtes stammt, an welchen die Produkte verabreicht werden, in Komplexmedien unter semianaeroben Wachstumsbedingungen kultiviert wird, wodurch die optimale Produktion von Metaboliten in der späten log-Phase und in der stationären Wachstumsphase auftritt, und daß die Metabolite aus dem Wachstumsmedium, in welches sie während der Kuitivierung freigesetzt werden, gewonnen werden.

3. Präparate, enthaltend als Wirkstoffkomponente hochmolekulargewichtige, proteinhaltige Metabolite, welche in dem Kultur-Überstand von Stämmen des Lactobacillus crispatus, Lactobacillus fermentum oder Lactobacillus murinus gefunden werden, vorzugsweise abstammend vom Gastrointestinaltrakt der Tiere, in welche die Präparate verabreicht werden, und wahlweise pharmazeutisch annehmbare Bestandteile, wobei die Metabolite die folgenden Charakteristika aufweisen:
a) sie haben Molekulargewichte von mehr als 8.000:
b) sie sind wärmeempfindlich;
c) sie sind gegenüber Säurepuffern empfindlich;
d) sie binden an enteropathogene E. coli-Stämme, wenn sie wachstumsinhibierend sind, und an den Mucus, wenn sie haftungsinhibierend sind;
e) sie haben in Gegenwart von Milchsäure verstärkte Aktivität;
f) ihre Produktion wird begünstigt durch Wachstum in Komplexmedien, im Gegensatz zu halbdefiniertem Medium;
g) ihre Produktion ist begünstigt unter semianaeroben Wachstumsbedingungen;
h) ihre Produktion ist optimal in der späten log-Phase und der stationären Wachstumsphase der Lactobacilli;
i) sie werden in das Wachstumsmedium freigesetzt.

## Revendications

1. Produits pour inhiber l'adhérence d'agents pathogènes aux muqueuses épithéliales gastro-intestinales, ou pour inhiber la croissance et/ou la survie d'agents pathogènes chez des animaux incluant les humains, caractérisés en ce qu'ils consistent en métabolites protéiques de haut poids moléculaire trouvés dans le surnageant de culture de souches de *Lactobacillus crispatus, Lactobacillus fermentum* ou *Lactobacillus murinus,* provenant de préférence des voies gastro-intestinales de l'hôte auquel les produits seront administrés, lesquels métabolites ont les caractéristiques suivantes :
(a) ils ont un poids moléculaire supérieur à 8.000;
(b) ils sont sensibles à la chaleur;
(c) ils sont sensibles aux tampons acides;
(d) ils se lient à des souches de *E.coli* entéropathogènes lorsqu'ils inhibent la croissance et aux muqueuses lorsqu'ils inhibent l'adhérence;
(e) ils présentent une plus grande activité en présence d'acide lactique;
(f) leur production est activée par une croissance dans des milieux complexes plutôt que dans un milieu semi-défini;
(g) leur production est activée dans des conditions de croissance semi-anaérobies;
(h) leur production est optimale dans la phase log tardive et la phase stationnaire de croissance des lactobacilles;
(i) ils sont libérés dans le milieu de croissance.

2. Procédé de production de produits pour inhiber l'adhérence d'agents pathogènes aux muqueuses épithéliales gastro-intestinales, ou pour inhiber la croissance et/ou la survie d'agents pathogènes chez des animaux incluant les humains, lesquels produits sont des métabolites protéiques de haut poids moléculaire produits par des souches de *Lactobacillus crispatus, Lactobacillus fermentum* ou *Lactobacillus murinus,* provenant de préférence des voies gastro-intestinales de l'hôte auquel les produits seront administrés, lesquels métabolites ont les caractéristiques suivantes :
(a) ils ont un poids moléculaire supérieur à 8.000;
(b) ils sont sensibles à la chaleur;
(c) ils sont sensibles aux tampons acides;
(d) ils se lient à des souches de *E.coli* entéropathogènes lorsqu'ils inhibent la croissance et aux muqueuses lorsqu'ils inhibent l'adhérence;
(e) ils présentent une plus grande activité en présence d'acide lactique;
(f) leur production est activée par une croissance dans des milieux complexes plutôt que dans un milieu semi-défini;
(g) leur production est activée dans des conditions de croissance semi-anaérobies;
(h) leur production est optimale dans la phase log tardive et la phase stationnaire de croissance des lactobacilles;
(i) ils sont libérés dans le milieu de croissance.
caractérisé en ce qu'une souche de *Lactobacillus* provenant de préférence des voies gastro-intestinales de l'hôte auquel les produits seront administrés, est cultivée dans des milieux complexes dans des conditions de croissance semi-anaérobies, de telle sorte que la production optimale de métabolites apparaît dans la phase log tardive et dans la phase stationnaire de croissance, et que les métabolites sont récupérés à partir du milieu de croissance dans lequel ils sont libérés pendant la culture.

3. Préparations contenant en tant qu'un composant actif, des métabolites protéiques de haut poids moléculaire trouvés dans le surnageant de culture de souches de *Lactobacillus crispatus, Lactobacillus fermentum* ou *Lactobacillus murinus,* provenant de préférence des voies gastro-intestinales de l'hôte auquel les préparations seront administrés, et éventuellement des constituants acceptables du point de vue pharmaceutique, lesquels métabolites ont les caractéristiques suivantes :
(a) ils ont un poids moléculaire supérieur à 8.000;
(b) ils sont sensibles à la chaleur;
(c) ils sont sensibles aux tampons acides;
(d) ils se lient à des souches de *E.coli* entéropathogènes lorsqu'ils inhibent la croissance et aux muqueuses lorsqu'ils inhibent l'adhérence;
(e) ils présentent une plus grande activité en présence d'acide lactique;
(f) leur production est activée par une croissance dans des milieux complexes plutôt que dans un milieu semi-défini;
(g) leur production est activée dans des conditions de croissance semi-anaérobies;
(h) leur production est optimale dans la phase log tardive et la phase stationnaire de croissance des lactobacilles;
(i) ils sont libérés dans le milieu de croissance.
